# EUROPEAN PATENT APPLICATION

(11) **EP 3 417 760 A1**
(43) Date of publication of application: **26.12.2018**
(21) Application number: 18185215.3
(22) Date of filing: 04.10.2013
(51) Int. Cl.: A61B 1/00

(54) **SEMICONDUCTOR APPARATUS, AND MANUFACTURING METHOD OF SEMICONDUCTOR APPARATUS**

(30) Priority: 23.10.2012 JP 2012233984; 12.11.2012 JP 2012248676
(62) Divisional of application: 13849748.2
(71) Applicant: OLYMPUS CORPORATION, Hachioji-shi Tokyo 192-8507 (JP)
(72) Inventor: YOSHIDA, Kazuhiro, Hachioji-shi, Tokyo 192-8507 (JP); NAKAYAMA, Takashi, Hachioji-shi, Tokyo 192-8507 (JP)
(74) Representative: Winter, Brandl, Fürniss, Hübner, Röss, Kaiser, Polte - Partnerschaft mbB

(57) **Abstract**

A semiconductor apparatus capable of being disposed in a narrow space and having high reliability is provided, and a manufacturing method of the semiconductor apparatus. The semiconductor apparatus includes: a semiconductor device chip having a first primary surface and a second primary surface; a wiring board mounted on the second primary surface of the semiconductor device chip and bent such that entirety of the wiring board overlaps the semiconductor device chip when the semiconductor device chip is viewed in a plan view in a thickness direction of the semiconductor device chip; and a resin filled in a space between the second primary surface of the semiconductor device chip and a mounting surface of the wiring board to be mounted on the second primary surface, and positioned such that entirety of the resin overlaps the semiconductor device chip when the semiconductor device chip is viewed in a plan view in the thickness direction of the semiconductor device chip, wherein the resin protrudes from the space along a bending portion on an outer circumferential surface of the wiring board on which the mounting surface is formed in a direction apart from the second primary surface in the thickness direction.

## Description

### Technical Field

The present invention relates to an image pickup apparatus having an image pickup device chip, an endoscope provided with the image pickup apparatus, a semiconductor apparatus having a semiconductor chip and a manufacturing method of the semiconductor apparatus, and particularly relates to an image pickup apparatus having a wiring board for connecting an image pickup device chip and a signal cable, an endoscope provided with the image pickup apparatus, a semiconductor apparatus having a wiring board connected with a semiconductor device chip, and a manufacturing method of the semiconductor apparatus.

### Background Art

An image pickup apparatus having an image pickup device chip is used, for example, by being disposed at a distal end portion of an endoscope. Regarding the distal end portion of the endoscope, there has been a considerable problem of thinning a diameter thereof in order to reduce pain on an examinee.

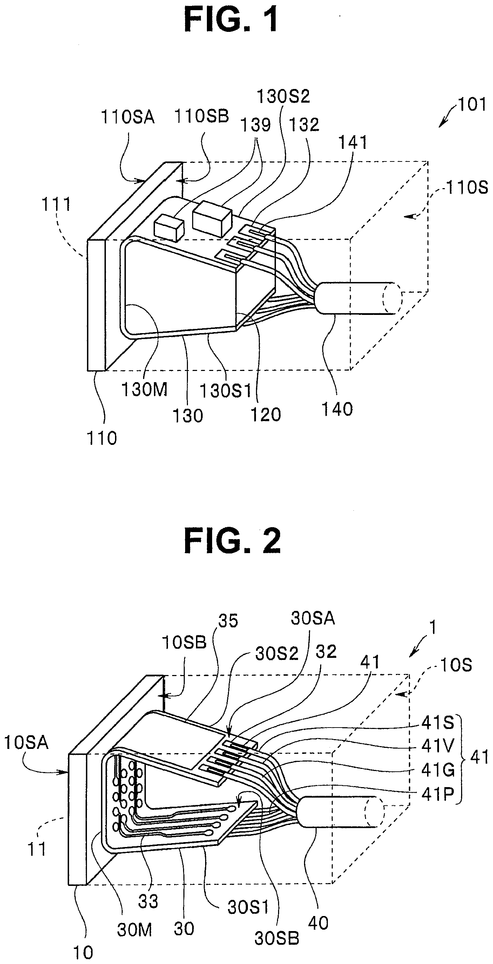

As shown in FIG. 1, in Japanese Patent Laid-Open Publication No. 2011-217887, there is described an image pickup apparatus 101 having an image pickup device chip 110, a block 120 which is a heat radiation member, a wiring board 130 on which electrical parts 139 are mounted, and a signal cable 140. The image pickup device chip 110 has in image pickup unit 111 on an obverse surface 110SA and a plurality of junction terminals (not shown) on a reverse surface 110SB. The junction terminals are joined to junction electrodes (not shown) of the wiring board 130. The junction electrodes are connected with terminal electrodes 132, which are joined to lead wires 141 of the signal cable 140, through wirings (not shown).

The wiring board 130 is comprised of a central portion 130M which is joined to the image pickup device chip 110 and extending portions 130S1 and 130S2 which extend from the central portion 130M on respective sides thereof. The extending portions 130S1 and 130S2 are bent inward. Therefore, the wiring board 130 is housed in an interior 110S of an extended space of a projected plane (within the projected plane) of the image pickup device chip 110. In the image pickup apparatus 101, heat generated by the image pickup device chip 110 is transmitted to the block 120 through the wiring board 130.

However, in the image pickup apparatus 101, there has been a fear that a temperature of the image pickup device chip 110 rises to deteriorate images if a heat transmitting function of the wiring board 130, which is located between the image pickup device chip 110 and the block 120, is insufficient.

It is noted that in Japanese Patent Laid-Open Publication No. 2010-199352, there is described a circuit board provided with a signal pattern, a power supply pattern and a heat radiation pattern. The heat radiation pattern is positioned at a region other than regions of the signal pattern and the power supply pattern and is electrically connected with the power supply pattern.

However, in the above circuit board, there is a fear that a heat amount that can be radiated from the heat radiation pattern is insufficient in a case where the circuit board is used in an image pickup apparatus which generates a large amount of heat, etc., and it has not been easy to apply the circuit board, as it is, to an ultra-small image pickup apparatus that is disposed at a distal end portion of an endoscope which has importance in thinning a diameter thereof.

Further, in Japanese Patent Laid-Open Publication No. 2012-38920, there is disclosed a semiconductor apparatus in which a flexible substrate (flexible wiring board) is connected to a semiconductor substrate, then a connection portion is filled with resin, and after the resin is hardened, the flexible substrate is bent to thereby prevent a failure by stress.

However, in the above semiconductor apparatus, there is a fear that the connection portion is tend to be disconnected if a load exerted on the connection portion and the resin is excessively large when the substrate is bent, to lower connection reliability.

### Summary of Invention

### Technical Problem

An object of the present invention is to provide an image pickup apparatus capable of being disposed in a narrow space and having an excellent heat radiation characteristic, an endoscope provided with the image pickup apparatus, a semiconductor apparatus capable of being disposed in a narrow space and having high reliability, and a manufacturing method of the semiconductor apparatus.

### Means for Solving the Problem

An image pickup apparatus according to an aspect of the present invention includes: an image pickup device chip that has an image pickup unit on an obverse surface and junction terminals on a reverse surface, the junction terminals being connected with the image pickup unit via through wirings; a signal cable having lead wires connected with the image pickup unit; and a wiring board that is constituted by a central portion and a plurality of extending portions extending from the central portion, and includes junction electrodes formed at the central portion and joined to the junction terminals, terminal electrodes formed at the extending portions and connected with the lead wires, wirings that connect the junction electrodes and the terminal electrodes, and a heat transmission pattern formed in a region where the junction electrodes, the terminal electrodes and the wirings are not formed, the extending portions being bent and thereby the wiring board being arranged within a projected plane of the image pickup device chip.

An endoscope according to another aspect of the present invention includes: an insertion portion having a distal end portion at which an image pickup apparatus is disposed, the image pickup apparatus including: an image pickup device chip that has an image pickup unit on an obverse surface and junction terminals on a reverse surface, the junction terminals being connected with the image pickup unit via through wirings; a signal cable having lead wires connected with the image pickup unit; and a wiring board that is constituted by a central portion and a plurality of extending portions extending from the central portion, and includes junction electrodes formed at the central portion and joined to the junction terminals, terminal electrodes formed at the extending portions and connected with the lead wires, wirings that connect the junction electrodes and the terminal electrodes, and a heat transmission pattern formed in a region where the junction electrodes, the terminal electrodes and the wirings are not formed, the extending portions being bent and thereby the wiring board being arranged within a projected plane of the image pickup device chip; an operation portion disposed on a distal end side of the insertion portion; and a universal cord extending from the operation portion.

A semiconductor apparatus according to another aspect of the present invention includes: a semiconductor device chip having a first primary surface and a second primary surface; a wiring board mounted on the second primary surface of the semiconductor device chip and bent such that entirety of the wiring board overlaps the semiconductor device chip when the semiconductor device chip is viewed in a plan view in a thickness direction of the semiconductor device chip; and a resin filled in a space between the second primary surface of the semiconductor device chip and a mounting surface of the wiring board to be mounted on the second primary surface, and positioned such that entirety of the resin overlaps the semiconductor device chip when the semiconductor device chip is viewed in a plan view in the thickness direction of the semiconductor device chip, wherein the resin protrudes from the space along a bending portion on an outer circumferential surface of the wiring board on which the mounting surface is formed in a direction apart from the second primary surface in the thickness direction.

A manufacturing method of a semiconductor apparatus according to another aspect of the present invention, includes: a wiring board mounting step of mounting a second primary surface opposite to a first primary surface of a semiconductor device chip on a wiring board; a jig attaching step of attaching a fixing jig to a surface opposite to a mounting surface of the semiconductor device chip, the second primary surface being mounted on the mounting surface; a wiring board bending step of positioning the wiring board on the semiconductor device chip such that entirety of the wiring board overlaps the semiconductor device chip when the semiconductor device chip is viewed in a plan view in a thickness direction of the semiconductor device chip connecting the first primary surface and the second primary surface by bending the wiring board, and maintaining a bent state of the wiring board by the fixing jig; and a resin filling step of filling a resin in a space between the second primary surface of the semiconductor device chip and the mounting surface of the wiring board to thereby position the resin such that entirety of the resin overlaps the semiconductor device chip when the semiconductor device chip is viewed in a plan view in the thickness direction, and hardening the resin, wherein in the resin filling step by filling the space with the resin of a filling amount not smaller than a first amount by which the resin is filled at 100% in the space, and not larger than a second amount by which the resin is filled at 100% in a set space between the mounting surface and the second primary surface when the mounting surface is extended to an outer periphery of the semiconductor chip, the resin protrudes from the space in a direction apart from the second primary surface in the thickness direction along a bending portion of the wiring board.

### Advantageous Effects of Invention

According to embodiments of the present invention, an image pickup apparatus capable of being disposed in a narrow space and having an excellent heat radiation characteristic, an endoscope provided with the image pickup apparatus, a semiconductor apparatus capable of being disposed in a narrow space and having high reliability, and a manufacturing method of the semiconductor apparatus can be provided.

### Brief Description of the Drawings

FIG. 1 is a perspective view of a conventional image pickup apparatus;
FIG. 2 is a perspective view of an image pickup apparatus according to a first embodiment;
FIG. 3 is a cross sectional view of the image pickup apparatus according to the first embodiment;
FIG. 4 is a plan view of a reverse surface of an image pickup device chip of the image pickup apparatus according to the first embodiment;
FIG. 5 is an exploded sectional view for explaining a manufacturing method of the image pickup apparatus according to the first embodiment;
FIG. 6 is a plan view of a first primary surface of a wiring board of the image pickup apparatus according to the first embodiment;
FIG. 7 is a plan view of a second primary surface of the wiring board of the image pickup apparatus according to the first embodiment;
FIG. 8 is a plan view of a first primary surface of a wiring board of an image pickup apparatus according to a second embodiment;
FIG. 9 is a plan view of a second primary surface of the wiring board of the image pickup apparatus according to the second embodiment;
FIG. 10 is a plan view of a first primary surface of a wiring board of an image pickup apparatus according to a third embodiment;
FIG. 11 is a plan view of a second primary surface of the wiring board of the image pickup apparatus according to the third embodiment;
FIG. 12 is a plan view of a first primary surface of a wiring board of an image pickup apparatus according to a fourth embodiment;
FIG. 13 is a plan view of a second primary surface of the wiring board of the image pickup apparatus according to the fourth embodiment;
FIG. 14 is a cross sectional view of the image pickup apparatus according to the fourth embodiment;
FIG. 15 is a cross sectional view of an image pickup apparatus according to a fifth embodiment;
FIG. 16 is a cross sectional view of an image pickup apparatus according to a sixth embodiment;
FIG. 17 is an external view of an endoscope according to a seventh embodiment;
FIG. 18 is a configuration view of a semiconductor apparatus according to an eighth embodiment;
FIG. 19 is an enlarged view of a region surrounded by XIX in FIG. 18 in the semiconductor apparatus according to the eighth embodiment;
FIG. 20 is a view showing a wiring board and a semiconductor device chip of the semiconductor apparatus according to the eighth embodiment when the wiring board is developed and viewed in a XX direction in FIG 18;
FIG. 21 is a view schematically showing a space of a joining portion of semiconductor apparatus according to the eighth embodiment;
FIG. 22 is a view showing a wiring board mounting step of a manufacturing method of the semiconductor apparatus according to the eighth embodiment;
FIG. 23 is a view showing a jig attaching step, the jig being attached, of the manufacturing method of the semiconductor apparatus according to the eighth embodiment;
FIG. 24 is a view showing a wiring board bending step of the manufacturing method of the semiconductor apparatus according to the eighth embodiment;
FIG. 25 is a view showing a resin filling step of the manufacturing method of the semiconductor apparatus according to the eighth embodiment;
FIG. 26 is a view showing a jig attaching step in the manufacturing method of the semiconductor apparatus according to modified example 1 of the eighth embodiment;
FIG. 27 is a view showing a wiring board bending step in the manufacturing method of the semiconductor apparatus according to the modified example 1 of the eighth embodiment;
FIG. 28 is a view showing a resin applying step in the manufacturing method of the semiconductor apparatus according to the modified example 1 of the eighth embodiment;
FIG. 29 is a view showing a semiconductor device chip mounting step in the manufacturing method of the semiconductor apparatus according to the modified example 1 of the eighth embodiment;
FIG. 30 is a configuration view of a semiconductor apparatus according to modified example 2 of the eighth embodiment;
FIG. 31 is an exploded perspective view of a wiring board and a semiconductor device chip of a semiconductor apparatus according to modified example 3 of the eighth embodiment;
FIG. 32 is a perspective view showing a state in which the semiconductor device chip is mounted at a center of the wiring board of the semiconductor apparatus according to the modified example 3 of the eighth embodiment;
FIG. 33 is a perspective view showing a state in which four regions of the wiring board of the semiconductor apparatus are bent according to the modified example 3 of the eighth embodiment;
FIG. 34 is a configuration view of a semiconductor apparatus according to a ninth embodiment;
FIG. 35 is a view showing a reinforcing resin filling step of a manufacturing method of the semiconductor apparatus according to the ninth embodiment;
FIG. 36 is a configuration view of a semiconductor apparatus according to a tenth embodiment;
FIG. 37 is a view showing an example of using the semiconductor apparatus according to the eighth embodiment as an image pickup apparatus; and
FIG. 38 is a cross sectional view of a semiconductor apparatus according to an eleventh embodiment.

### Best Mode for Carrying Out the Invention

### <First Embodiment>

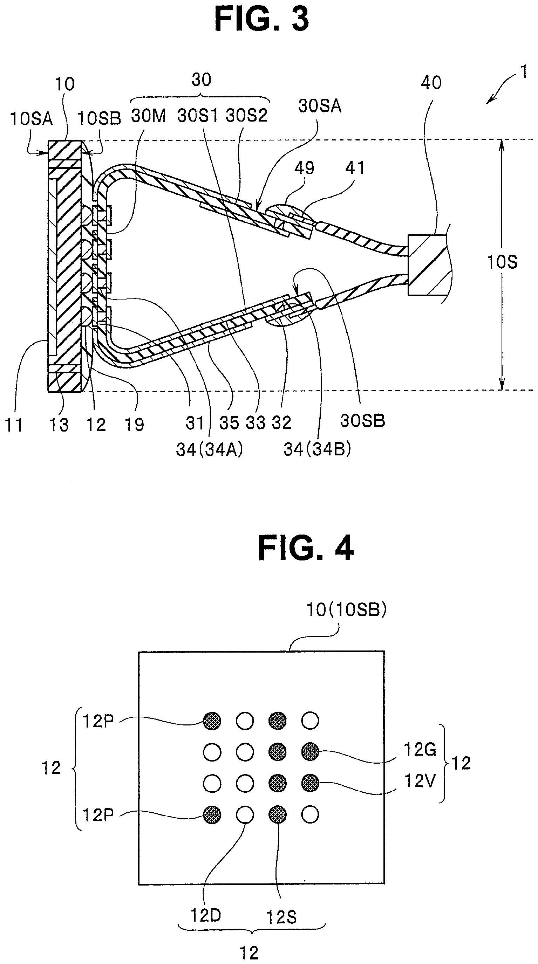

An image pickup apparatus 1 according to a first embodiment, as shown in FIG. 2 and FIG. 3, is similar to the conventional image pickup apparatus 101 already described. That is, the image pickup apparatus 1 is provided with an image pickup device chip 10, a wiring board 30, a signal cable (hereinafter also simply referred to as "cable") 40.

The wiring board 30 is constituted by a central portion 30M which is joined to the image pickup device chip 10 on a side of a first primary surface 30SA, and extending portions 30S 1, 30S2 which are provided to extend from the central portion 30M on respective sides thereof. The extending portions 20S1, 30S2 are bent toward a second primary surface 30SB. Therefore, the wiring board 30 is located to be enclosed in an interior 10S of an extended space of a projected plane (within the projected plane) of the image pickup device chip 10.

On an obverse surface 10SA of the image pickup device chip 10 which is comprised of a semiconductor, there is formed an image pickup unit 11 of a CMOS device, for example, which is a solid-state image pickup device. The image pickup device chip 10 has a plurality of junction terminals 12, which are connected with the image pickup unit 11 on the obverse surface 10SA via respective through wirings 13, on a reverse surface 10SB. It is noted that wirings for connecting the image pickup unit 11 and the through wirings 13 are formed on the obverse surface 10SA but these wirings are not shown.

The image pickup device chip 10 are manufactured, for example, using a known semiconductor process, by forming a number of image pickup units 11, through wirings 13, etc. on a silicone substrate, and then cutting the substrate. Therefore, the image pickup device chip 10 has an approximately rectangular shape in a plan view. It is preferable that dimensions of the image pickup device chip 10 in the plan view, in other words, an area of the primary planes is small in order to reduce a diameter of the image pickup apparatus 1.

Here, as shown in FIG. 3 and FIG. 4, the image pickup device chip 10 of the image pickup apparatus 1 has sixteen junction terminals 12 having the same shape in appearance. However, eight terminals out of the above terminals are dummy junction terminals 12D which are not connected with the image pickup unit 11. In FIG. 4, the dummy junction terminals 12D are indicated by white circles. That is, the eight terminals are the dummy junction terminals 12D, excluding eight terminals of a junction terminal 12V for supplying power to the image pickup unit 11, two junction terminals 12P for supplying a pulse signal to the image pickup unit 11, four junction terminals 12S for transmitting and receiving signals to and from the image pickup unit 11, and a junction terminal 12G for causing the image pickup unit 11 to have the ground potential.

The junction terminal 12V is connected with a lead wire 41V of the cable 40 which supplies the power, the junction terminals 12P are connected with lead wires 41P which supply the pulse signal, the junction terminals 12S are connected with lead wires 41S which transmit and receive the signals, and the junction terminal 12G is connected with a lead wire 41G of the ground potential. (Hereinafter, the lead wires 41V, 41P, 41S and 41G are also referred to simply as "lead wires 41".)

The number of junction terminals 12 and the number of dummy junction terminals 12D and arrangements thereof are selected in accordance with specifications of the image pickup apparatus. Further, functions and the like of the junction terminals 12 are not limited to the above configuration. For example, there may be provided one junction terminal 12P or a plurality of junction terminals 12G. (Hereinafter, the dummy junction terminals 12D, and the junction terminals 12V, 12P, 12S and 12G are also referred to simply as "junction terminals 12").

Terminal electrodes 32 of the wiring board 30 and lead wires 41 of the cable 40 are joined by solder 49, for example. It is noted that the lead wire 41G of the ground potential may be a shield wire covering circumferences of the other lead wires. Further, each of the lead wires may have a shield wire. Particularly, it is preferable that the lead wires 41P and the lead wires 41S, which tend to cause a noise or be influenced by a noise, are shield lines. It is not necessary that the cable 40 is arranged in the interior 10S of the extended space of the projected plane (hereinafter referred to as "within the projected plane") over the entire length of the cable, and it is sufficient that at least a joining portion to the wiring board 30 is arranged in the interior 10S of the projecting plane.

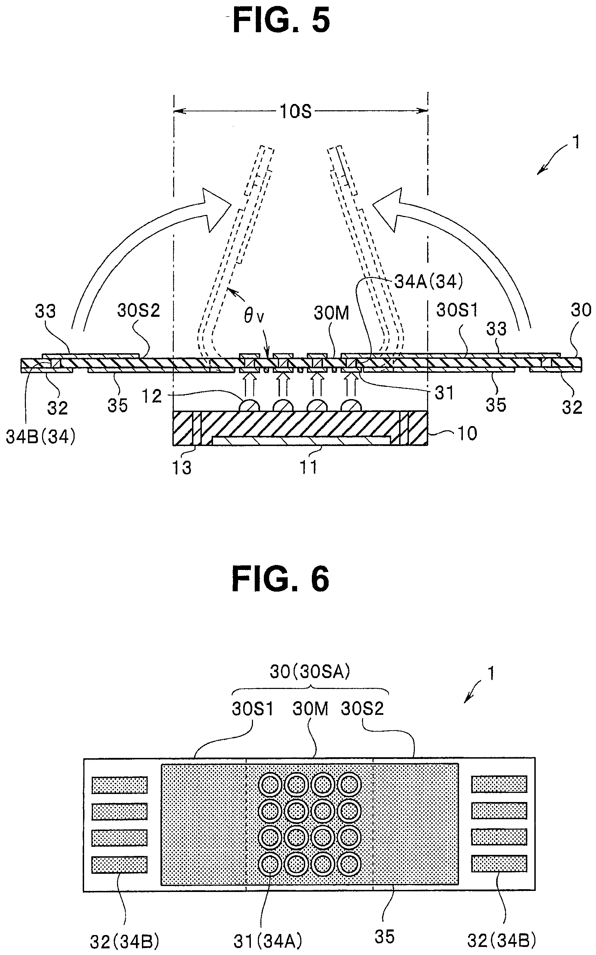

As shown in FIG. 5 and FIG. 6, the wiring board 30 is arranged in the interior 10S of the projected plane of the image pickup device chip 10 by bending the extending portions 30S1 and 30S2 with the first primary surface 30SA located outside (with the second primary surface 30SB located inside). A bending angle θv is preferably set to an angle of 90-50 degrees and particularly preferably set to an angle of 75-55 degrees, and is for example an angle of 65 degrees. If the bending angle is set within the above range, the joining portion of the wiring board 30 and the cable 40 can be arranged in the interior 10S of the projected plane of the image pickup device chip 10. It is noted that there is a case where an angle of 90 degrees is most preferable as the bending angle θv, as described later.

Since the wiring board 30 is one flexible wiring board, boundaries of the central portion 30M and the extending portions 30S1, 30S2 are not clearly defined. Besides, it may be configured that at least bent portions of the wiring board are flexible and the wiring board is a rigid flexible wiring board in which part or all of the central portion 30M and extending portions 30S1 and 30S2 is constituted by a rigid substrate.

The wiring board 30 has junction electrodes 31, which are joined to the junction terminals 12 of the image pickup device chip 10, on the first primary surface 30SA in the central portion 30M. The wiring board 30 has sixteen junction electrodes 31 in appearance but eight electrodes of these are dummy junction electrodes 31D which are not connected with the image pickup unit 11.

The eight dummy junction electrodes 31D are respectively joined to the dummy junction terminals 12D. Therefore, the image pickup apparatus 1 is configured such that the image pickup device chip 10 and the wiring board 30 are easily jointed in parallel and joining strength is large. Further, heat generated by the image pickup device chip 10 can be transmitted to the wiring board 30 more efficiently.

Besides, as shown in FIG. 3, it is preferable that a space between the image pickup device chip 10 and the wiring board 30 is sealed by a sealing resin 19. A gold bump, a solder ball, an ACP (anisotropic conductive plastic), an ACF (anisotropic conductive film) or the like is used for joining the junction terminals 12 and the junction electrodes 31.

The junction electrodes 31 on the first primary surface 30SA are connected with wirings 33 on the second primary surface 30SB via through wirings 34A, and the wirings 33 are connected with terminal electrodes 32 on the first primary surface 30SA of the extending portions 30S1, 30S2 via through wirings 34B. Hereinafter, the through wirings 34A and 34B are referred to as through wirings 34. It is noted that the dummy junction electrodes 31D may have connection portions on the second primary surface 30SB via through wirings. However, the dummy junction electrodes 31D are not connected with any electrode other than the ground potential wire (lead wire 41G).

Since the wiring board 30 is a both-side wiring board, the junction electrodes 31 and the terminal electrodes 32 are connected with one another through the wires 33 and the through wires 34A, 34B, but in a case of a one-side wiring board, through wirings for connection of the junction electrodes 31 and the terminal electrodes 32 are unnecessary. In contrast, a multi-layer wiring board having three wiring layers or more may be used. Further, an electronic part 13may be implemented in the middle of the wiring 33. As the electronic part, a necessary part is selected from among a chip condenser, a chip resister, a signal processing IC, a driver IC, a power supply IC, a diode, a coil, a lead switch, etc.

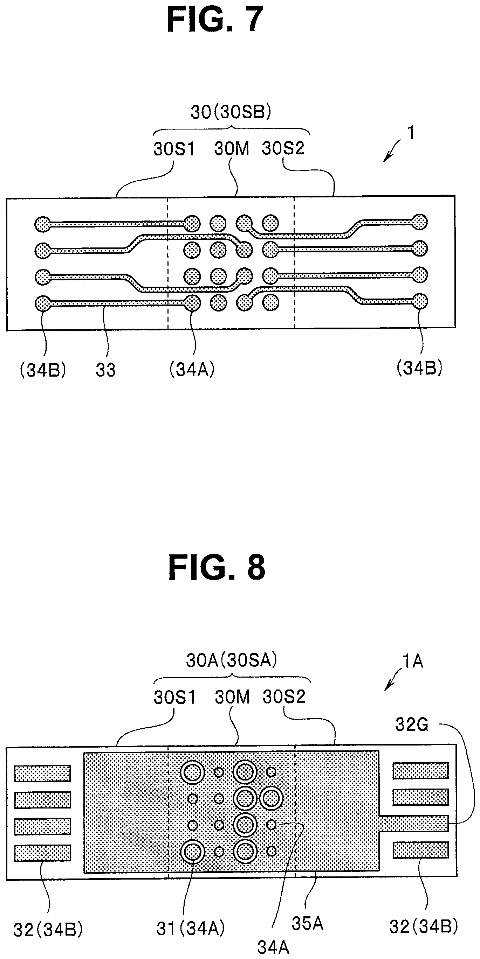

Further, as shown in FIG. 6 and FIG. 7, the wiring board 30 has a heat transmission pattern 35, which is formed in a region where the junction electrodes 31 and the terminal electrodes 32 are not formed, on the first primary surface 30SA. In the wiring board 30 exemplified in FIG. 6, the heat transmission pattern 35 is formed from the central portion 30M to the extending portions 30S1, 30S2, to surround the junction electrodes 31. The heat transmission pattern 35 transmits heat generated by the image pickup unit 11 to a proximal end portion side.

As shown in FIG. 7, no heat transmission pattern is formed on the second primary surface 30SB, but a second heat transmission pattern may be formed in a region where the wirings 33 are not formed.

The junction electrodes 31, the terminal electrodes 32 and the heat transmission pattern 35 are formed, for example, by pattern-etching a conductive film which is formed on entirety of the first primary surface 30SA. That is, the conductive film is removed by etching in a frame shape so that the junction electrodes 31 and the heat transmission pattern 35 are not electrically connected. As a matter of course, the heat transmission pattern 35, etc. may be formed by a plating method. Further, the heat transmission pattern 35 may be formed into a film shape to be thicker than the junction electrodes 31, the terminal electrodes 32, etc.

It is preferable that an area of the heat transmission pattern 35 is large and it is particularly preferable that the area is not less than 50% and not more than 90% of an area of the first primary surface 30SA. If the area is not less than the above lower limit, an effect of improving a heat radiation characteristic is remarkable and if the area is not more than the above upper limit, a necessary area for the junction electrodes 31 and the terminal electrodes 32 can be secured.

Besides, it may be configured such that the terminal electrodes 32 and the wirings 33 are formed in at least one of the extending portions 30S1 and 30S2, and the heat transmission pattern 35 is formed in at least one of the extending portions 30S1 and 30S2.

It is easy to reduce external dimensions of the image pickup apparatus 1 since the wiring board 30, etc. can be arranged in a predetermined narrow space. Therefore, the image pickup apparatus 1 is capable of being disposed stably in a narrow space. Further, the heat generated by the image pickup device chip 10 is transmitted to the proximal end side through the heat transmission pattern 35. Thus, the image pickup apparatus 1 is excellent in the heat radiation characteristic.

In addition, since the dummy junction terminals 12D of the image pickup device chip 10 and the dummy junction electrodes 31D of the wiring board 30 are joined to one another, the image pickup apparatus 1 is further excellent in the heat radiation characteristic.

### <Second Embodiment>

Next, an image pickup apparatus 1A according to a second embodiment will be described. The image pickup apparatus 1A is similar to the image pickup apparatus 1 and therefore the same reference signs are assigned to the same elements and the description thereof is omitted.

As shown in FIG. 8, in a wiring board 30A of the image pickup apparatus 1A, a terminal electrode 32G, which is connected with the lead wire 41G of the ground potential of the cable 40, is integrated with a heat transmission pattern 35A. Further, the dummy junction electrodes 31D are also integrated with the heat transmission pattern 35A. That is, the dummy junction electrodes 31D and the heat transmission pattern 35A are connected with each other. It is noted that a first primary surface 30SA of the wiring board 30A is covered with a resin layer (not shown) except for joining regions with the junction terminals 12 of the image pickup device chip 10.

The image pickup apparatus 1A has the effects of the image pickup apparatus 1 and further since the heat transmission pattern 35A having a wide area is at the ground potential, noise radiation caused by transmission and reception of signals is prevented and the apparatus is less influenced by a noise from outside. Further, the image pickup apparatus 1A has a higher heat radiation effect than the image pickup apparatus 1 since the dummy joining electrodes 31D are connected with the heat transmission pattern 35A.

### <Third Embodiment>

Next, an image pickup apparatus 1B according to a third embodiment will be described. The image pickup apparatus 1B is similar to the image pickup apparatuses 1 and 1A, and therefore the same reference signs are assigned to the same elements and the description thereof is omitted.

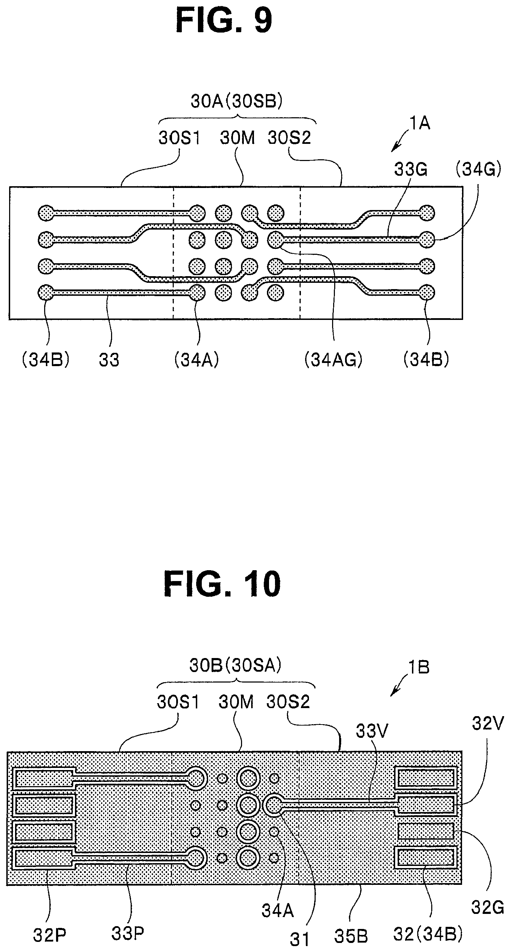

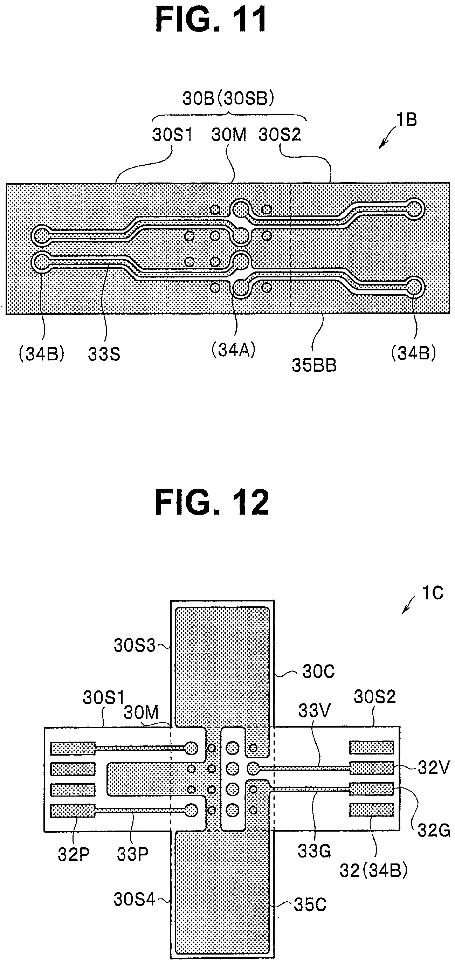

As shown in FIG. 10 and FIG. 11, a wiring board 30B of the image pickup apparatus 1B includes, on the first primary surface 30SA, a power supply wiring 33V for supplying power to the image pickup unit 11, terminal electrodes 32P and pulse wirings 33P which are joined to the lead wires 41P for supplying a clock signal as the pulse signal and a heat transmission pattern 35B, and includes on the second primary surface 30SB, terminal electrodes 32S and signal wirings 33S which are joined to the lead wires 41S for transmitting and receiving signals to and from the image pickup apparatus 11.

The image pickup apparatus 1B has the effects of the image pickup apparatuses 1 and 1A, and further since the pulse wirings 33P and the signal wirings 33S are formed on different primary surfaces of the wiring board 30B, deterioration of a received image caused by interference of the signals is prevented.

Besides, as long as the pulse wirings 33P and the signal wirings 33S are respectively formed on the different primary surfaces, the number of wirings, arrangements thereof, etc. are not limited to the above configuration and are selected according to specifications of the image pickup apparatus.

Further, since the wiring board 30B has a second heat transmission pattern 35BB, which is at the ground potential, further on the second primary surface 30SB, the image pickup apparatus 1B has a higher heat radiation effect and a higher shield effect than the image pickup apparatuses 1 and 1A.

### <Fourth Embodiment>

Next, an image pickup apparatus 1C according to a fourth embodiment will be described. The image pickup apparatus 1C is similar to the image pickup apparatuses 1, 1A and 1B, and therefore the same reference signs are assigned to the same elements and the description thereof is omitted.

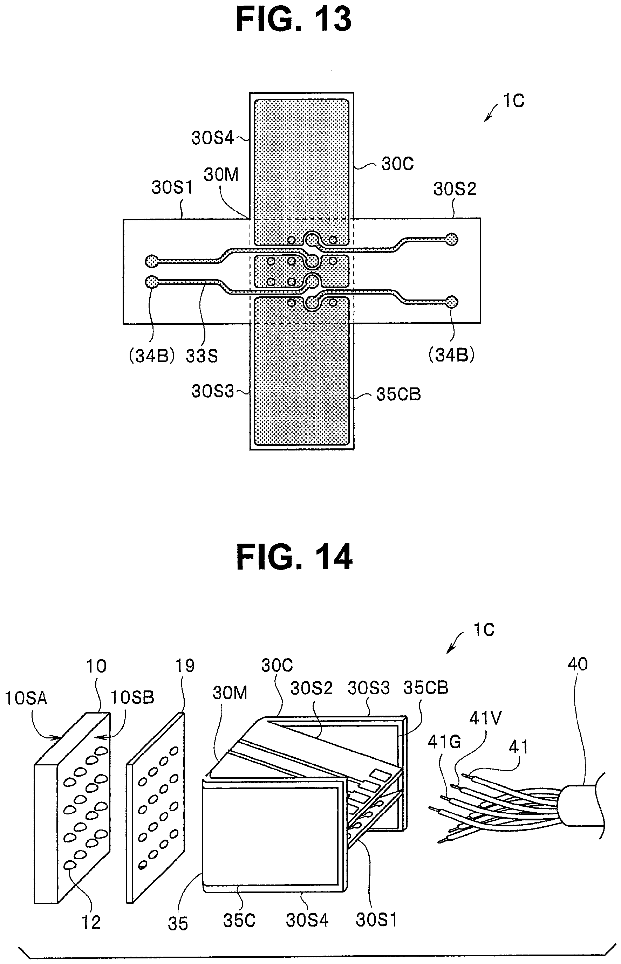

As shown in FIG. 12 and FIG. 13, a wiring board 30C of the image pickup apparatus 1C includes a central portion 30M having a substantially squire shape, and four extending portions 30S1-30S4 which have substantially rectangular shapes and extend from the central portion 30M in directions orthogonal with each other.

As shown in FIG. 14, the four extending portions 30S1-30S4 are all bent inward and therefore the wiring board 30C is arranged in the interior 10S of the projected surface of the image pickup device chip 10.

It is noted that a bending angle θv of the extending portions 30S1 and 30S2 is set to an angle of 75-55 degrees, and a bending angle θv of the extending portions 30S3 and 30S4 is set to an angle of approximately 90 degrees.

The image pickup apparatus 1C has the effects of the image pickup apparatuses 1, 1A and 1B, and further since heat transmission patterns 35C and 35CB are formed on the extending portions 30S3 and 30S4, respectively, the apparatus has a better heat radiation characteristic. In particular, since the wirings 33, etc. are not formed on the extending portions 30S3 and 30S4, heat transmission patterns having wider areas can be formed.

Besides, it is a matter of course that the same effects as the image pickup apparatus 1C can be obtained in an image pickup apparatus which is provided with a wiring board having three extending portions extending in directions orthogonal to each other.

### <Fifth Embodiment>

Next, an image pickup apparatus 1D according to a fifth embodiment will be described. The image pickup apparatus 1D is similar to the image pickup apparatuses 1, 1A, 1B and 1C, and therefore the same reference signs are assigned to the same elements and the description thereof is omitted.

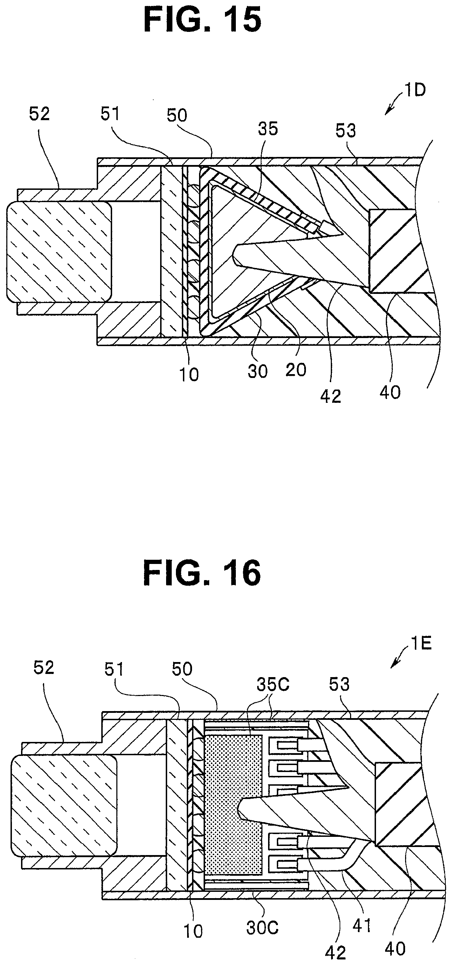

As shown in FIG. 15, the image pickup apparatus 1D further includes a cover glass 51 joined to the obverse surface 10SA of the image pickup device chip 10, an optical unit 52 constituted by a lens and a support body thereof, and an external cylinder portion 50 made of a metal. It is noted that FIG. 16 is a schematic view composed by sectional views along different planes so as to make the description easy, and the wirings 33, etc. are not shown. Further, it is usual that the optical unit 52 is constituted by optical members such as a plurality of lens, and a fixing member and a support body thereof, but the optical unit is schematically depicted.

A distal end portion of the cable 40, the image pickup device chip 10, the cover glass 51 and the wiring board 30 are housed in the external cylinder portion 50. That is, an inside dimension of the external cylinder portion 50 is substantially equal to the projected plane of the image pickup device chip 10. Inside the external cylinder portion 50, a non-conductive resin 53 having high thermal conductivity such as a silicone resin is filled.

Further, the image pickup apparatus 1D is provided with a block 20 which is a heat radiation member with which the extending portions are in contact. The block 20 is in contact with the second primary surface 30SB of the wiring board 30, and has a function as a fixing member for arranging the wiring board 30 in a predetermined space, i.e. in the interior 10S of the projected plane of the image pickup device chip 10. Further, the block 20 makes a joining work of the cable 40 to the wiring board 30 easy since the block 20 retains the wiring board 30 stably.

The block 20 also has a function as a reinforcing member for retaining the image pickup device chip 10 and the wiring board 30 to be integrated therewith and increasing mechanical strength. Specifically, there is a fear that the image pickup device chip 10 is deformed or broken by an external force since the image pickup device chip is formed of a silicone substrate, for example. However, the mechanical strength of the image pickup device chip 10 is increased by the block 20 being joined through the wiring board 30. Similarly, the strength of the wiring board 30 is increased as being joined to the block 20 although flexibility is substantially lost.

Further, the image pickup apparatus 1D is configured such that the wiring board 30 is always in a bent state at a predetermined angle without using a special jig or the like when bending the board since the wiring board 30 is fixed in a state of being in contact with the block 20. That is, the image pickup apparatus 1 is configured such that the wiring board 30, etc. are capable of being easily disposed in a predetermined narrow space.

It is noted that, in the present specification, "contact" includes not only a case of direct contact without any other member but also a case of being joined through a thin junction film. For example, a silicone resin having high heat conductivity can be used preferably as the junction film.

Further, the cable 40 of the image pickup apparatus 1D has a shield wire 42 which covers the plurality of lead wires 41 and is made of a mesh-like metal. The shield wire 42 is joined to the block 20 and the exterior cylinder portion 50. Therefore, heat of the block 20 and the exterior cylinder portion 50 is transmitted to the proximal end side efficiently through the shield wire 42 having high heat conductivity.

The image pickup apparatus 1D has the effects of the image pickup apparatuses 1-1C, and further is easy to manufacture and excellent in the heat radiation characteristic.

### <Sixth Embodiment>

Next, an image pickup apparatus IE according to a sixth embodiment will be described. The image pickup apparatus IE is similar to the image pickup apparatuses 1-1D and therefore the same reference signs are assigned to the same elements and the description thereof is omitted.

As shown in FIG. 16, the image pickup apparatus IE has the wiring board 30C with the extending portions in four directions similarly to the image pickup apparatus 1C. Further, a part of the heat transmission pattern 35C is in contact with an inner surface of the exterior cylinder portion 50. It is noted that FIG. 16 is a schematic view similar to FIG. 15 and the wirings 33, etc. are not shown.

In the image pickup apparatus IE, the heat generated by the image pickup unit 11 is transmitted to the exterior cylinder portion 50 efficiently through the heat transmission pattern 35C. Therefore, the image pickup apparatus IE has the effects of the image pickup apparatuses 1-1D, and further is excellent in the heat radiation characteristic.

### <Seventh Embodiment>

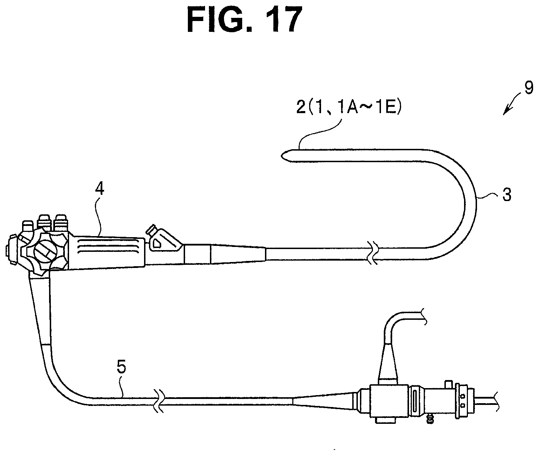

Next, an endoscope 9 according to a seventh embodiment will be described. As shown in FIG. 17, the endoscope 9 has the image pickup apparatus 1, 1A-1E at a distal end portion 2 of an insertion portion 3. Further, the endoscope 9 is provided with an operation portion 4 disposed at a proximal end side of the insertion portion 3 and a universal cord 5 extending form the operation portion 4.

At the operation portion 4, there are disposed various kinds of switches and the like to be operated by a surgeon while grasping the operation portion. The cable 40 of the image pickup apparatus 1 is inserted through the insertion portion and the universal cord 5 and connected with a main body unit (not shown), which performs image processing, etc., via a connector disposed at a proximal end portion of the universal cord.

Since the endoscope 9 is provided with the image pickup apparatus 1, 1A-1E, which is capable of being disposed in a narrow space and excellent in the heat radiation characteristic, at the distal end portion, the endoscope has the distal end portion with a small diameter and is excellent in thermal stability.

### <Eighth Embodiment>

### <Configuration of a semiconductor apparatus>

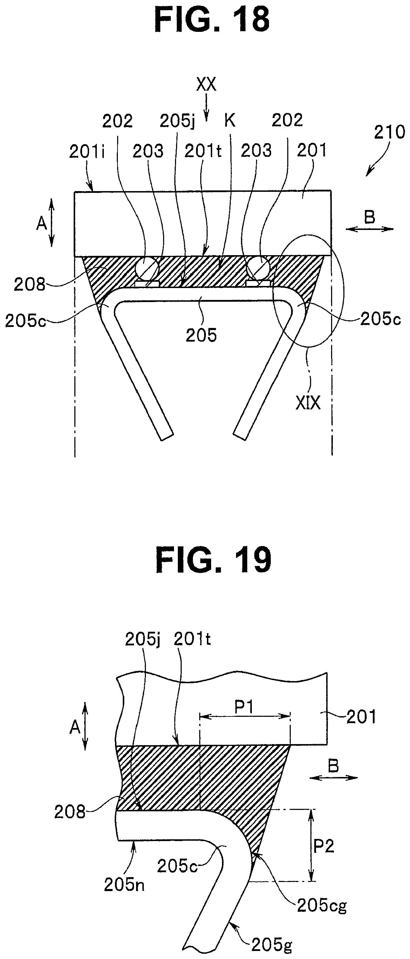

As shown in FIG. 18 and FIG. 19, a semiconductor apparatus 210 of an eighth embodiment is provided with a semiconductor apparatus (hereinafter referred to as "semiconductor chip") 201 and a flexible substrate (hereinafter, referred to as "wiring board") 205.

Connection terminals 202 are provided on a second primary surface (a rear surface 201t) of the semiconductor chip 201, which is opposite to a first primary surface (a front surface 201i) thereof. Substrate electrodes 203 provided on a mounting surface 205i, as described later, of the wiring board 205 are electrically connected to the connection terminals 202. Since the wiring board 205 is bent, entirety of the wiring board 205 overlaps the semiconductor chip 201 when the semiconductor chip 201 is viewed in a plan view in a thickness direction A connecting the front surface 201i and the rear surface 201t.

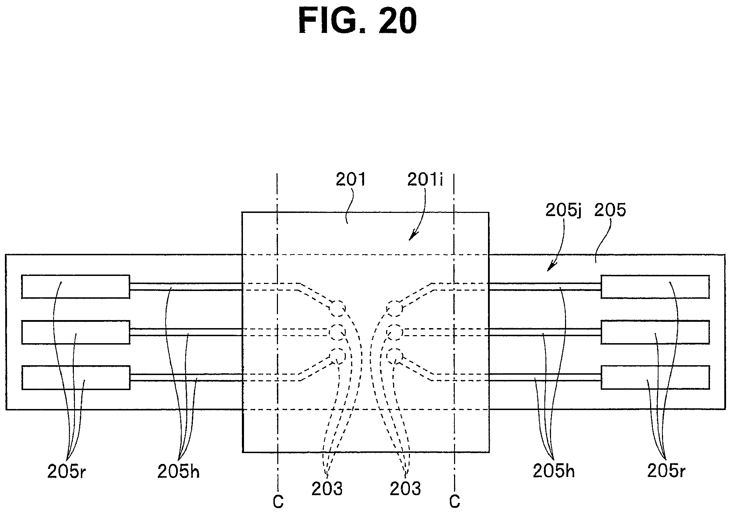

The wiring board 205 having a substrate of a flexible resin such as polyimide is bent, as shown in FIG. 20, between an outer periphery of the semiconductor chip 201 and the substrate electrodes 203 into a circular arc shape, for example, at two positions along the one-dot chain lines C so that a bending angle is not larger than 90 degrees. Therefore, the wiring board 205 is disposed in a direction (hereinafter referred to as "rearward direction") apart from the rear surface 201t in the thickness direction A of the semiconductor chip 201 so that the entirety of the wiring board 205 overlaps the semiconductor chip 201 when the semiconductor chip 201 is viewed in a plan view in the thickness direction A.

That is, the wiring board 205 has bending portions 205c formed at two positions. A region between the two bending portions 205c on an outer circumferential surface 205g of the wiring board 205 which is parallel to the rear surface (second primary surface) 201t of the semiconductor chip 201 is referred to as "mounting surface 205j".

It is noted that, as shown in FIG. 20, on the outer circumferential surface 205g of the wiring board 205, the substrate electrodes 203, connection electrodes 205r to which signal cables (not shown) are electrically connected, a wiring pattern 205h which electrically connects the connection electrodes 205r and the substrate electrodes 203 are formed.

Here, if the bending portions 205c are bent at an angle greater than 90 degrees, when an electronic part is implemented on the outer circumferential surface 205g of the wiring board 205 or signal cables are electrically connected to the connection electrodes 205r, the electronic part or the signal cables protrudes outside of an outer shape of the semiconductor chip 201, to make the semiconductor apparatus 210 large in size. Besides, it is preferable that the bending angle of the bending portions 205 is set to be an acute angle smaller than 90 degrees.

A resin 208 is a filling resin for underfilling, for example. The resin 208 is filled at least in a space K between the rear surface 201f of the semiconductor chip 201 and the mounting surface 205j, rearward of the rear end surface 201f, and entirety of the resin 208 overlaps the semiconductor chip 201 when the semiconductor chip 201 is viewed in a plan view in the thickness direction A.

Further, as shown in FIG. 18 and FIG. 19, the resin 208 protrudes outside of the mounting surface 205j from the space K by a length P1 in an extent not to be protruded outside of the outer shape of the semiconductor chip 201 in a horizontal direction B orthogonal to the thickness direction A. It is noted that "protruding outside" means a state of extending outside from a predetermined range.

Furthermore, the resin 208 protrudes from the space K rearward of the mounting surface 205j in the thickness direction A along the bending portions 205c on the outer circumferential surface 205g of the wiring board 205 by a length P2. That is, the resin 208 protrudes from the space K to outer circumferential surfaces 205cg of the bending portions 205c. It is noted that the resin 208 protruded to the outer circumferential surfaces 205cg of the bending portions 205c is positioned not to protrude outside of the outer shape of the semiconductor chip 201 in the horizontal direction B.

Besides, it is preferable that the outer circumferential surfaces 205cg of the bending portions 205c, to which the resin 208 adheres, are subjected to hydrophilic processing by plasma cleaning or the like or formed to have high surface roughness so that adhesiveness of the protruded resin 208 with respect to the outer circumferential surfaces 205cg of the bending portions 205c is improved.

It is noted that the resin 208 may contain carbon particles or pigment or the like and may contain filler or the like. If the carbon particles or the pigment is contained, a light shield property can be improved and if the filler having high heat conductivity is contained, a heat radiation property can be improved.

### <Manufacturing method of a semiconductor apparatus>

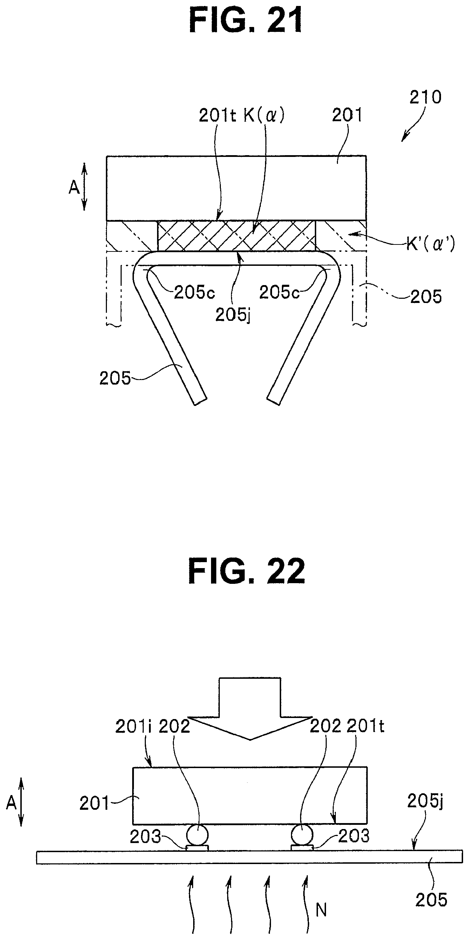

Next, the manufacturing method of a semiconductor apparatus will be described using FIGS. 21-25.

### <Mounting step>

As shown in FIG. 22, a wiring board mounting step for electrically connecting the substrate electrodes 203 of the wiring board 205 in an unbent state (a planar state) to the connection terminals 202 provided on the rear surface 201t of the semiconductor chip 201 is performed. The electric connection of the substrate electrodes 203 to the connection terminals 202 is performed by applying heat H from a side of the wiring board 205.

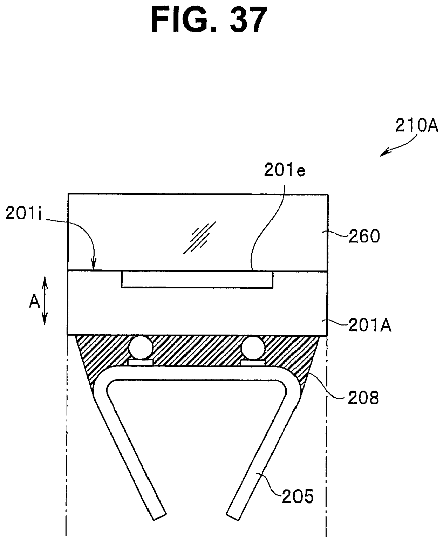

The application of the heat N is performed from the side of the wiring board 205 because it is difficult to apply the heat N to a connection region from a side of the semiconductor chip 201 in a case where a cover glass 260 (see FIG. 37), as described later, is attached to the front surface 201i of the semiconductor chip 201.

### <Jig attaching step>

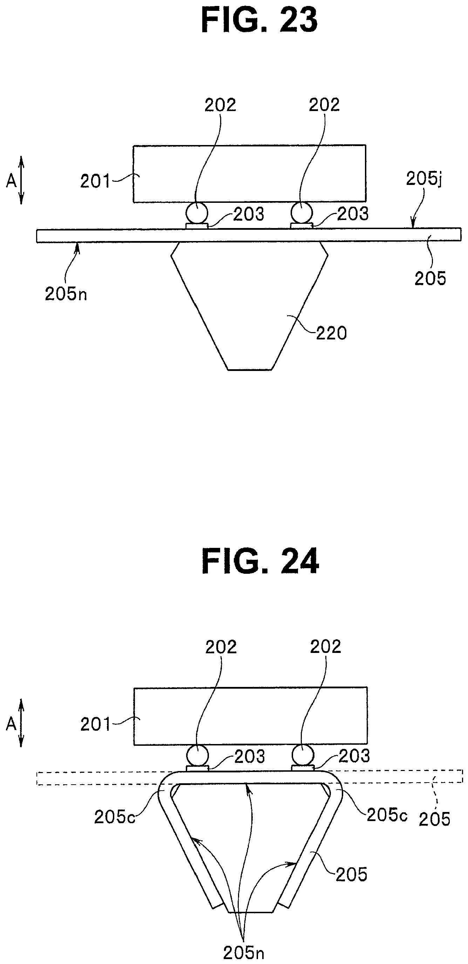

As shown in FIG. 23, a jig attaching step is performed for attaching a fixing jig 20 on an inner circumferential surface 205n opposite to the mounting surface 205j of the wiring board 205 at a position where the jig 20 overlaps the semiconductor chip 201 when the semiconductor chip 201 is viewed in a plan view in the thickness direction A.

### <Bending step>

As shown in FIG. 24, a wiring board bending step is performed for positioning the wiring board 205 so that the entirety of the wiring board 205 overlaps the semiconductor chip 201 when the semiconductor chip 201 is viewed in a plan view in the thickness direction A by bending the wiring board 205 at two positions, in particular, along the lines C as shown in FIG. 20 at an angle not greater than 90 degrees, and mechanically holding the bent shape of the wiring board 205 by the fixing jig 220. It is noted that the inner circumferential surface 205n of the wiring board 205 is in contact with an outer circumferential surface of the fixing jig 220.

### <Resin filling step>

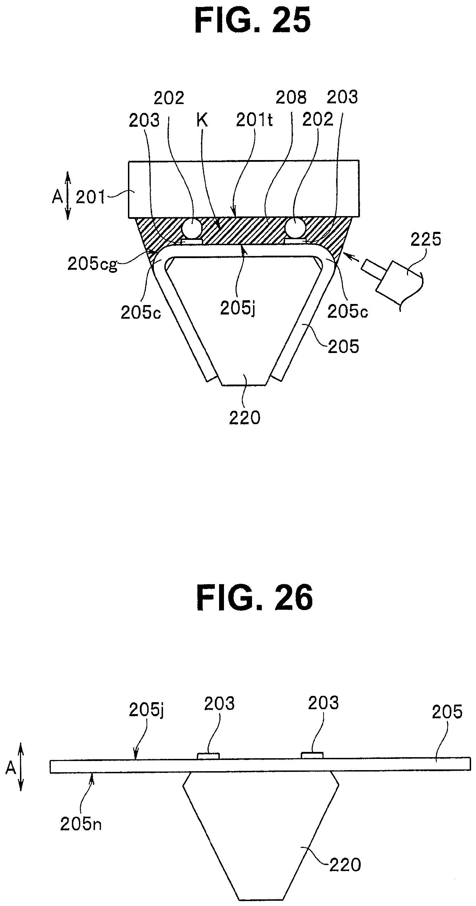

As shown in FIG. 25, a resin filling step is performed for filling the resin 208 in at least the space K between the rear surface 201t of the semiconductor chip 201 and the mounting surface 205j of the wiring board 205. In the resin filling step, the resin 208 is filled so that the entirety of the resin 208 overlaps the semiconductor chip 201 rearward of the rear surface 201t when the semiconductor chip 201 is viewed in a plan view in the thickness direction A, and the resin 208 is hardened by an oven or the like.

The resin 208 of a filling amount not smaller than a first amount α by which the resin 208 is filled at 100% in the space K, as shown in FIG. 21, and not larger than a second amount α' by which the resin 208 is filled at 100% in a set space K' between the rear surface 201t and the mounting surface 205j when it is extended to the outer periphery of the semiconductor chip 201, as shown by the two-dot chain line in FIG. 21, is filled at least in the space K from below the mounting surface 205j, particularly, from the vicinity of the bending portion 205c, using a dispenser 225, for example, as shown in FIG. 25.

Besides, by filling the first amount α of resin in the space M and then applying the resin 208 on the outer circumferential surfaces 205cg of the bending portions 205c in a separate step, protruded portions of the resin 208 having the length P2 along the bending portions 205c may be formed.

Consequently, the resin 208 protrudes from the space K in the thickness direction A rearward of the mounting surface 205j by the length P2 along the bending portions 205c of the wiring board 205.

### <Jig removing step>

After the resin filling step, a jig removing step of removing the fixing jig 220 from the wiring board 205 is performed and thereby the semiconductor apparatus 210 is manufactured.

In the present embodiment, the wiring board 205 is located rearward of the semiconductor chip 201 by the configuration that the substrate electrodes 203 are electrically connected to the connection terminals 202 on the rear surface 201t of the semiconductor chip 201, and further the wiring board 205 is located such that the entirety of the wiring board 205 overlaps the semiconductor chip 201 when the semiconductor chip 201 is viewed in a plan view in the thickness direction A by being bent at the two positions.

Further, the resin 208 filled in at least the space K between the mounting surface 205i of the wiring board 205 and the rear surface 201t of the semiconductor chip 201 is located to protrude from the space K to the outer circumferential surfaces 205cg of the bending portions 205c, and the resin 208 is filled such that the entirety of the resin also overlaps the semiconductor chip 201 when the semiconductor chip 201 is viewed in a plan view in the thickness direction A.

Therefore, since the wiring board 205 and the resin 208 do not protrude outward from the outer periphery of the semiconductor chip 201 in the horizontal direction B, the semiconductor apparatus 210 can be reduced in size.

Further, the resin 208 protruded from the space K to the outer circumferential surfaces 205cg of the bending portions 205c fixes the wiring board 205 firmly, and prevents an action of the wiring board 205 to return to the unbent state (which is sometimes called as a spring back), so that the bent state of the wiring board 205 can be maintained, and therefore the connection reliability of the substrate electrodes 203 with respect to the connection terminals 202 is improved.

Furthermore, in the manufacturing method of the semiconductor apparatus of the present embodiment, the resin 208 is filled in the space K after the wiring board 205 is bent. Therefore, a load is not exerted on the resin 208 when bending the wiring board 205 after the resin is hardened, and the wiring board 205 is easily positioned such that the entirety of the wiring board overlaps the semiconductor chip 201 when the semiconductor chip 201 is viewed in a plan view in the thickness direction A.

Further, the connection of the connection terminals 202 of the semiconductor chip 201 to the substrate electrodes 203 of the wiring board 205 can be performed easily by a general mounting method such as the known SMT (Surface Mount Technology).

As described above, according to the present embodiment, the wiring board 205 can be firmly fixed to the semiconductor chip 201 and the semiconductor apparatus 210 which is small in size and the manufacturing method of the semiconductor chip 201 can be provided by positioning the bent wiring board 205 within the outer periphery of the semiconductor chip 20.

### <Modified Example 1>

A manufacturing method according to modified method 1 will be described using FIGS. 26-29.

### <Jig attaching step>

As shown in FIG. 26, in the modified example 1, a jig attaching step is performed for attaching the fixing jig 220 on the inner circumferential surface 205n opposite to the mounting surface 205j of the wiring board 205.

### <Bending step>

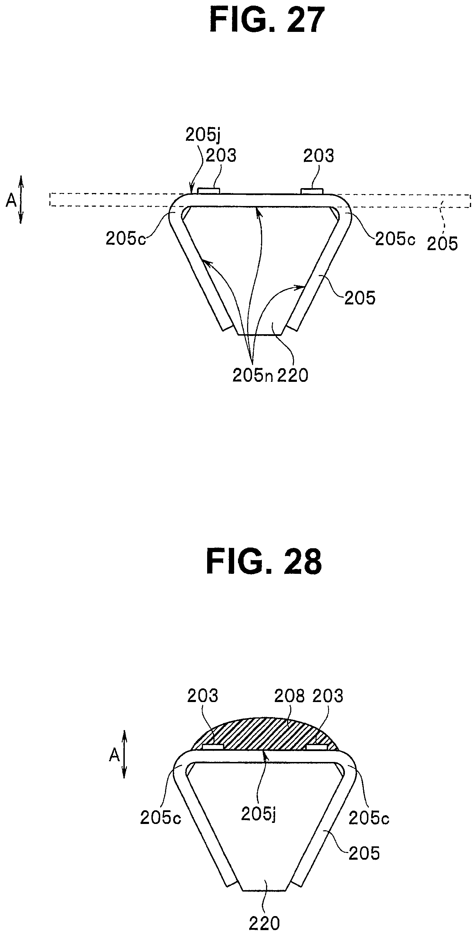

Next, as shown in FIG. 27, a wiring board bending step is performed for bending the wiring board 205 at two positions, in particular, along the lines C as shown in FIG. 20 at an angle not greater than 90 degrees, to thereby mechanically maintain the bent shape of the wiring board 205 by the fixing jig 220. It is noted that the inner circumferential surface 205n of the wiring board 205 is in contact with the outer circumferential surface of the fixing jig 220 after the bending.

### <Resin applying step>

As shown in FIG. 28, a resin applying step is performed for applying the resin 208 on the mounting surface 205j of the wiring board 205.

### <Mounting step>

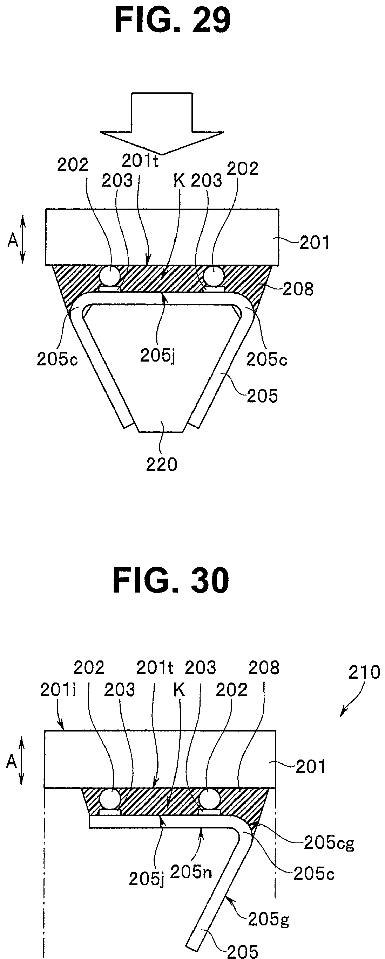

Thereafter, as shown in FIG. 29, a semiconductor device chip mounting step is performed for electrically connecting the connection terminals 202 of the semiconductor chip 201to the substrate electrodes 203 provided on the mounting surface 205j of the wiring board 205 while squashing the resin 208 by exerting a load thereto by the semiconductor chip 201, and thereby positioning the resin 208 and the wiring board 205 such that entireties of the resin 208 and the wiring board 205 overlap the semiconductor chip 201 rearward of the rear surface 201t in at least the space K when the semiconductor chip 201 is viewed in a plan view in the thickness direction A, and hardening the resin 208 by heating means until the mounting on the wiring board 205 is finished or after the mounting.

The resin 208 of a filling amount not smaller than the first amount α by which the resin 208 is filled at 100% in the space K, as shown in FIG. 21, and not larger than the second amount α' by which the resin 208 is filled at 100% in the set space K' between the rear surface 201t and the mounting surface 205j when it is extended to the outer periphery of the semiconductor chip 201, as shown by the two-dot chain line in FIG. 21, is applied on the mounting surface 205j, as shown in FIG. 28.

Consequently, the resin 208 protrudes from the space K rearward of the mounting surface 205j in the thickness direction A by the length P2 along the bending portions 205c of the wiring board 205 after the semiconductor device chip mounting step.

Besides, it may be configured such that, after applying the first amount α of resin on the mounting surface 205j, the substrate electrodes 203 are connected to the connection terminals 202, and then the resin 208 is further applied on the outer circumferential surfaces 205cg of the bending portions 205c to thereby form the protruded portions of the resin 208 having the length P2 along the bending portions 205c.

### <Jig removing step>

After the semiconductor device chip mounting step, a jig removing step of removing the fixing jig 220 from the wiring board 205 is performed and thereby the semiconductor apparatus 210 of the modified example 1 is manufactured.

In the modified example 1, since the substrate electrodes 203 are electrically connected to the connection terminals 202 of the semiconductor chip 201 after the wiring board 205 is bent, an effect that a bending stress exerted on the wiring board is reduced, and also the same effects as the embodiments such as the effect that a load is not exerted on the resin 208 in bending the wiring board 205 can be obtained.

### <Modified Example 2>

A modified example 2 will be described using FIG. 30.

In the eighth embodiment, the wiring board 205 is bent at the two positions, i.e. the two bending portions 205c are formed.

In contrast, as shown in FIG. 30, in the codified example 2, only one bending portion 205c is formed, and the wiring board 205 is positioned such that entirety of the wiring board 205 overlaps the semiconductor chip 201 rearward of the semiconductor chip 201 when the semiconductor chip 201 is viewed in a plan view in the thickness direction A in the same manner as the eighth embodiment.

It is noted that the resin 208 do not protrude outward from the outer periphery of the semiconductor chip 201 in the horizontal direction B, and the resin 208 protrudes from the space K to the outer circumferential surface 205cg of one bending portion 205c by the length P2.

### <Modified Example 3>

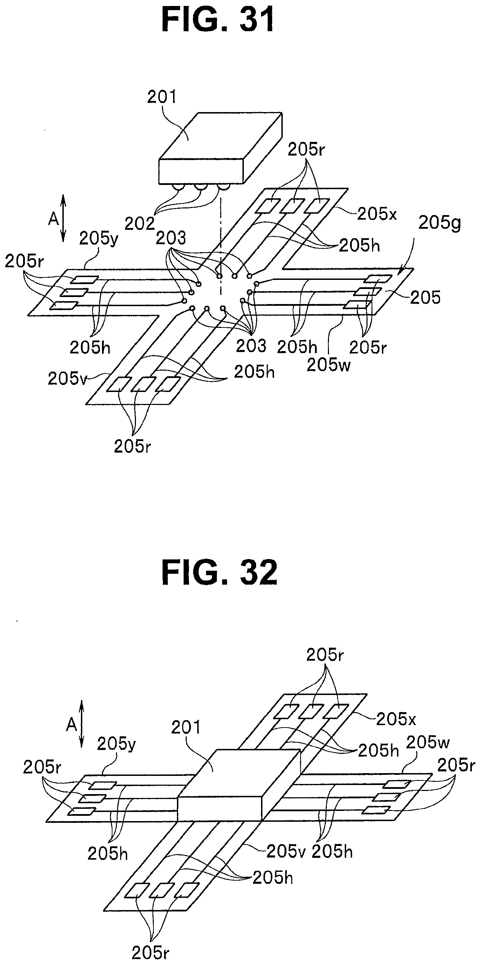

A modified example 3 will be described using FIGS. 31-33.

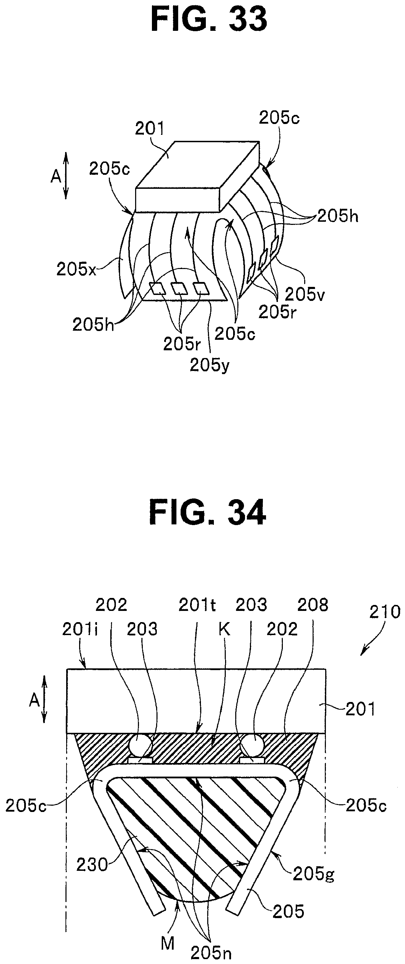

As shown in FIG. 33, the wiring board 205 of the modified example 3 has four bending portions 205c formed thereon.

As shown in FIG. 31, the connection terminals 202 of the semiconductor chip 201 are electrically connected to the substrate electrodes 203 which are exposed on the outer circumferential surface 205g of the wiring board 205 in a cross shape having four regions 205v, 205w, 205x and 205y at a central portion at which the four regions 205v, 205w, 205x and 205y intersect with each other, and then as shown in FIG. 33, the regions 205v, 205w, 205x and 205y are bent at an angle not greater than 90 degrees, so that the bending portions 205c are formed at four positions.

In the third modified example also, the wiring board 205 is positioned such that the entirety of the wiring board 205 overlaps the semiconductor chip 201 rearward of the semiconductor chip 201 when the semiconductor chip 201 is viewed in a plan view in the thickness direction A, and further the resin 208 do not protrude outward from the outer periphery of the semiconductor chip 201 in the horizontal direction B, and protrudes from the space K to the outer circumferential surfaces 205cg of the four bending portions 205c by the length P2.

### <Ninth Embodiment>

The present embodiment differs from the eighth embodiment in comparison therewith in that a reinforcing resin is fixed on the inner circumferential surface of the wiring board and that a step of filling the reinforcing resin on the inner circumferential surface of the wiring board is provided. Therefore, only different points will be described, the same reference signs are assigned to the same elements in the eighth embodiment and the description thereof is omitted.

As shown in FIG. 34, in the semiconductor apparatus 210 of the present embodiment, a reinforcing resin 230 for fixing a bent shape of the wiring board 205 is fixed to an inner circumferential surface 205n of the wiring board 205. That is, the reinforcing resin 230 is provided in a space M inside the wiring board 205. It is noted that the reinforcing resin 230 may be made of the same material as the resin 208 or a different material may be used.

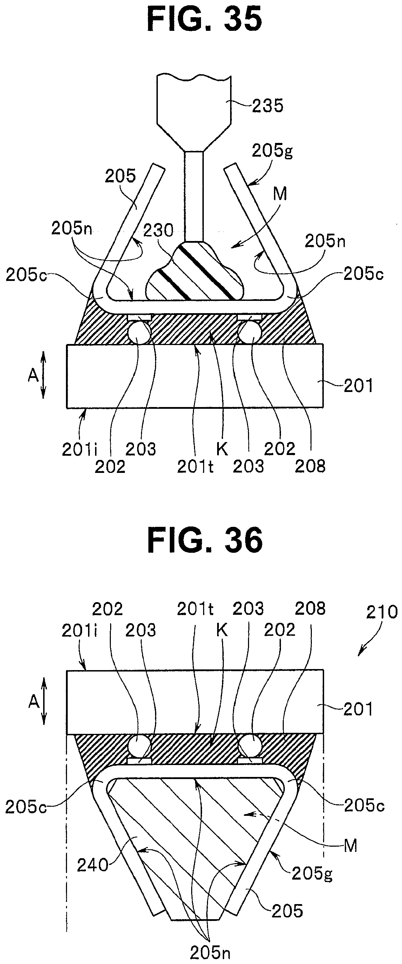

A manufacturing method of the semiconductor apparatus of the present embodiment is shown using FIG. 35. FIG. 35 is a view showing a reinforcing resin filling step of filling the reinforcing resin in the space inside the wiring board in FIG. 34.

In the present embodiment, the reinforcing resin filling step is performed in which the semiconductor apparatus 210, which is obtained after the jig removing step, is turned upside down (reversed in the thickness direction A) and then the reinforcing resin 230 for fixing the bent shape of the wiring board 205 is filled in the inner space M from an opening of the wiring board 205 by a dispenser 235 or the like.

Consequently, the reinforcing resin 230 is fixed on the inner circumferential surface 205n of the wiring board 205.

According to the present embodiment, in addition to the effects of the eighth embodiment, the strength of the semiconductor apparatus 210 can be more improved since the bent shape of the wiring board 205 is further reinforced by the reinforcing resin 230.

### <Tenth Embodiment>

The configuration of the semiconductor apparatus of the present embodiment differs from the eighth embodiment in comparison therewith in that a heat radiation member is fixed to the inner circumferential surface of the wiring board, and that a step of fixing the heat radiation member to the inner circumferential surface of the wiring board is provided. Therefore, only different points will be described, the same reference signs are assigned to the same elements in the eighth embodiment and the description thereof is omitted.

As shown in FIG. 36, in the semiconductor apparatus 210 of the present embodiment, a heat radiation member 240 that fixes the bent shape of the wiring board 205 and radiates heat transmitted from the semiconductor chip 201 through the wiring board 205 is fixed to the inner circumferential surface 205n of the wiring board 205. That is, the heat radiation member 240 is provided in the space M inside the wiring board 205. It is noted that as the heat radiation member 240, inorganic material such as SUS, aluminum, ceramics or resin material may be used.

It is noted that, according to the manufacturing method of the present embodiment, the heat radiation member 240 having the same shape as the fixing jig 220 is used instead of the fixing jig 220, in the jig attaching step of the eighth embodiment.

Besides, in the present embodiment, since the heat radiation member 240 is left in the space M, the jig removing step described in the eighth embodiment is unnecessary.

According to the present embodiment, the number of manufacturing steps can be reduced since the jig removing step is unnecessary, the bent shape of the wiring board 205 is further reinforced, and the heat radiation property of the semiconductor apparatus 210 can be improved by the heat radiation member 240.

### <Image pickup apparatus>

Here, the semiconductor apparatuses of the eighth-tenth embodiments are used, for example, as image pickup apparatuses. For example, FIG. 37 is a view showing an example of using the semiconductor apparatus 210 according to the eighth embodiment as an image pickup apparatus 210A. The semiconductor chip is an image pickup device chip 201A on which a light receiving portion 201e is formed. A cover glass 260 for covering the light receiving portion 201e is attached to a front surface 201i of the image pickup device chip 201A.

By applying the semiconductor apparatuses of the eighth-tenth embodiments to the image pickup apparatuses, the image pickup apparatuses are downsized since the wiring boards 205 and resins 208 are positioned within the outer peripheries of the semiconductor apparatuses 210, and are preferably installed at distal ends of endoscopes which are required to be downsized and reduced in diameter.

Besides, it is a matter of course that the image pickup apparatuses may be provided in medical capsule endoscopes as well as medical or industrial endoscopes, and may be applied to mobile phones with cameras, or digital cameras. Further, the semiconductor apparatuses 210 are applicable to other apparatuses different from the image pickup apparatuses.

### <Eleventh Embodiment>

The image pickup apparatuses 1-1E, etc. of the first-seventh embodiments may be provided with the configurations of the semiconductor apparatuses of the eighth-tenth embodiments.

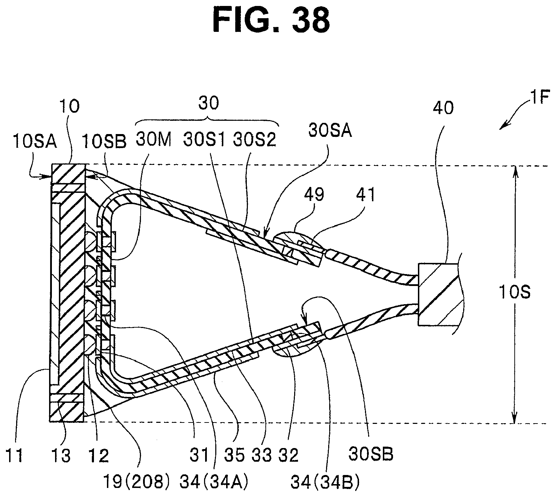

For example, an image pickup apparatus IF of the eleventh embodiment as shown in FIG. 38 includes an image pickup device chip that has an image pickup unit on an obverse surface and junction terminals on a reverse surface, the junction terminals being connected with the image pickup unit via through wirings; a signal cable having lead wires connected with the image pickup unit; a wiring board that is constituted by a central portion and a plurality of extending portions extending from the central portion, and includes junction electrodes formed at the central portion and joined to the junction terminals, terminal electrodes formed at the extending portions and connected with the lead wires, wirings that connect the junction electrodes and the terminal electrodes, and a heat transmission pattern formed in a region where the junction electrodes, the terminal electrodes and the wirings are not formed, the extending portions being bent and thereby the wiring board being arranged within a projected plane of the image pickup device chip; and a resin that is filled in a space between the semiconductor device chip and the wiring board, and protrudes from the space in a direction apart from a primary surface in a thickness direction with respect to the mounting surface of the wiring board along a bending portion on an outer circumferential surface of the wiring board on which the mounting surface is formed.

The image pickup apparatus IF has the effects of the image pickup apparatuses 1-1 E of the first-seventh embodiments and the semiconductor apparatus according to the eighth-tenth embodiments.

The present invention is not limited to the above-described embodiments, etc. and may be subjected to various changes, modifications, combinations and the like.

The present application is filed claiming the priority of Japanese Patent Application No. 2012-233984 filed in Japan on October 23, 2012 and Japanese Patent Application No. 2012-248676 filed in Japan on November 12, 2012, and the above described disclosure is incorporated by reference in the present description, claims and drawings.

## Claims

1. A semiconductor apparatus comprising:
a semiconductor device chip having a first primary surface and a second primary surface;
a wiring board mounted on the second primary surface of the semiconductor device chip and bent such that entirety of the wiring board overlaps the semiconductor device chip when the semiconductor device chip is viewed in a plan view in a thickness direction of the semiconductor device chip; and
a resin filled in a space between the second primary surface of the semiconductor device chip and a mounting surface of the wiring board to be mounted on the second primary surface, and positioned such that entirety of the resin overlaps the semiconductor device chip when the semiconductor device chip is viewed in a plan view in the thickness direction of the semiconductor device chip,
wherein the resin protrudes from the space along a bending portion on an outer circumferential surface of the wiring board on which the mounting surface is formed in a direction apart from the second primary surface in the thickness direction,
wherein a surface of the bending portion to which the resin adheres is subjected to hydrophilic processing.

2. The semiconductor apparatus according to claim 1,
wherein a reinforcing resin for fixing a bent shape of the wiring board is disposed on an inner circumferential surface of the wiring board.

3. The semiconductor apparatus according to claim 1 or 2,
a heat radiation member that fixes a bent shape of the wiring board and radiates heat transmitted from the semiconductor chip through the wiring board is disposed on an inner circumferential surface of the wiring board.

4. A manufacturing method of a semiconductor apparatus, comprising:
a jig attaching step of attaching a fixing jig to a surface opposite to a mounting surface of the wiring board on which a semiconductor device chip is mounted;
a wiring board bending step of bending the wiring board and maintaining a bent shape of the wiring board by the fixing jig;
a resin applying step of applying a resin on the mounting surface of the wiring board;
a semiconductor device chip mounting step of mounting a second primary surface opposite to a first primary surface of the semiconductor device chip on the mounting surface of the wiring board while squashing the resin, thereby positioning the bent wiring board and the resin so that entireties of the bent wiring board and the resin overlap the semiconductor chip in at least a space between the second primary surface of the semiconductor device chip and the mounting surface of the wiring board when the semiconductor chip is viewed in a plan view in a thickness direction of the semiconductor device chip connecting the first primary surface and the second primary surface, and hardening the resin,
wherein in the resin applying step by applying, in the space, the resin of a filling amount not smaller than a first amount by which the resin is filled at 100% in the space, and not larger than a second amount by which the resin is filled at 100% in a set space between the mounting surface and the second primary surface when the mounting surface is extended to an outer periphery of the semiconductor chip, the resin protrudes from the space in a direction apart from the second primary surface in the thickness direction with respect to the mounting surface along a bending portion of the wiring board.

5. A manufacturing method of a semiconductor apparatus according to claim 4,
wherein a surface of the bending portion to which the resin adheres is subjected to hydrophilic processing.

6. A manufacturing method of a semiconductor apparatus according to claim 4 or 5,
further comprising a jig removing step of removing the fixing jig from the wiring board after the semiconductor device chip mounting step.

7. The manufacturing method of a semiconductor apparatus according to claim 6,
further comprising a reinforcing resin filling step of filling a reinforcing resin for fixing the bent shape of the wiring board on an inner circumferential surface opposite to an outer circumferential surface of the wiring board.

8. The manufacturing method of a semiconductor apparatus according to claim 4 to 7,
wherein the fixing jig is a heat radiation member that fixes the bent shape of the wiring board and radiates heat transmitted from the semiconductor device chip through the wiring board.
